# EUROPEAN PATENT SPECIFICATION

(11) **EP 4 531 701 B1**
(45) Date of publication and mention of the grant of the patent: **18.11.2026**
(21) Application number: 23730607.1
(22) Date of filing: 02.06.2023
(51) Int. Cl.: A61B 10/00, A61B 5/097, A61B 5/08, G01N 33/497

(54) **METHOD AND SYSTEM FOR PREPARING A BREATH SAMPLE FOR ANALYSIS**
VERFAHREN UND SYSTEM ZUR VORBEREITUNG EINER ATEMPROBE ZUR ANALYSE
PROCÉDÉ ET SYSTÈME DE PRÉPARATION D'UN ÉCHANTILLON D'AIR EXPIRÉ POUR ANALYSE

(30) Priority: 03.06.2022 SE 2250675
(43) Date of publication of application: 09.04.2025
(73) Proprietor: Munkplast AB, 752 28 Uppsala (SE)
(72) Inventor: STAMBECK, Peter, 743 71 Björklinge (SE)
(74) Representative: Bergenstråhle & Partners AB
(86) International application number: PCT/SE2023/050549
(87) International publication number: WO 2023/234843

(56) References cited:
- WO-A1-2017/091134
- WO-A1-2021/061330
- SE-A1- 1 550 930
- US-A1- 2010 186 524
- US-A1- 2012 302 907
- US-A1- 2022 034 854

## Description

### Technical Field

The present disclosure relates generally to a method and system for preparing a breath sample for analysis. The breath sample has been taken to collect aerosol particles consisting mainly of surfactant formed or found in the alveoli of the lungs, such as biomarkers or exogenous compounds containing traces of drugs or other substances.

### Background Art

Human breath contains aerosol particles that are formed from the respiratory tract lining fluid covering the airways during normal breathing. Said particles have a size of between 0.1 and 2 µm, with an average size of between 0.3 and 0.8 µm. See article Characterization of Exhaled particles from the Healthy Human Lung, Journal of aerosol medicine and pulmonary drug delivery, Volume 23, Number 6, 2010 by Schwarz et al. The aerosol particles carry non-volatile components containing diagnostic information or biomarkers and are often studied as the breath condensate fraction. In this aerosol fraction, both lipids and peptides of endogenous origin have been demonstrated. It has also been discovered that exogenous compounds are present in the exhaled breath. Such exogenous compound may for example be drugs and narcotics. The respiratory tract lining fluid contain large quantities of antioxidants and surfactant. The surfactant phase is lipophilic and may represent a compartment for the exogenous compounds. Thus, exhaled breath can be used as a matrix for several types of analysis such as for example testing of a medical condition or a medical treatment procedure, abused drug testing or doping testing. It can also be used for medical research.

With the discovery of exogenous aerosol particles consisting mainly of surfactant present in exhaled breath, a need for new methods and devices for collecting and analyzing said surfactant aerosol particles in exhaled breath has arisen. For accurate analysis it is of importance that as many of the aerosol particles as possible is collected from a sample breath. Further, in some applications, such as for example testing for drug abuse or doping, the collection of particles is performed away from a lab environment.

WO 2017/001217 A1 discloses a collecting device having a labyrinth or zigzag-shaped flow path formed by baffles in an elongated housing which forces the exhaled flow of air to change direction multiple times. The change of direction of the airflow separates the heavier aerosol particles in the exhaled air from the air itself. The heavier particles continue in the original flow direction and collide with and attaches to the baffles or the inner wall of the housing, while the air changes direction and follow the labyrinth-shaped flow path.

WO 2017/091134 A1 discloses a portable sampling device for use with one or more collecting devices in accordance with WO 2017/001217 A1. After a sample has been proved by a user, the collecting device is removed from the sampling device and sent to e.g. a laboratory to extract the collected particles from the collecting device. To this end, the collecting device is inserted into a test tube or vial and an amount of a solvent called an eluent fluid is added to release the particles through the process of elution, i.e. washing. The eluent fluid is selected depending on the adsorption strength of the analyte (collected particles), the desired eluting power of the eluent fluid and the effect on the analyte so as not to destroy the particles. Typically, the eluent fluid is acetone, methanol, ethanol or water.

Eluent fluid can be expensive which drives cost in cases where a great number of samples are to be analyzed. Evaporating the resulting volume of eluate fluid is also time consuming, limiting the throughput and capacity of analysis. Thus, there is a need to find improved solutions which reduce the cost and time of analysis.

### Summary of Invention

An object of the present disclosure is to overcome the problems encountered by the available prior art as outlined above to achieve an improved method and system for preparing breath samples for analysis which reduces cost and time of analysis. The method and system for preparing a breath sample for analysis are described in the appended claims.

According to a first aspect of the present disclosure, there is provided a method for preparing a breath sample for analysis. The breath sample is collected using a device for collecting aerosol particles in an exhaled airflow. The device comprises a housing and at least four transverse baffles. The housing extends in a longitudinal direction between a first end with an inlet and a second end with an outlet. Additionally, the housing has an inner cross-section with a transverse width defined by an inner circumferential wall of the housing. The at least four transverse baffles are arranged at a distance from each other and extend substantially perpendicular to the inner wall, partly covering the inner cross section of the housing. The transverse baffles protrude from opposite sides of the inner wall of the housing to create a zigzag-shaped flow path from the inlet to the outlet.

The method for preparing the breath samples comprises the steps of placing said collecting device in a receptacle; then adding an eluent fluid onto the collecting device in the receptacle in a quantity smaller than or equal to 20 µl up to a total volume of approximately 30-60 µl and repeating the adding step one or more times, wherein each adding step is performed with a predetermined time delay; followed by agitating the receptable using a shaker; then centrifuging the receptacle using a centrifuge; and finally removing the collecting device from the receptacle.

By adding the eluent fluid stepwise and in small quantities, the amount of eluent fluid required for washing the collected particles from the inner walls and baffles of the collecting device can be reduced. At the same time, it was found that the eluent fluid will advance slowly across the inner walls and baffles of the collecting device to cover substantially all the interior surfaces where particles have been collected. This leads to a significant reduction in the preparation time of the breath sample for analysis since there is considerably less eluate to be evaporated after agitation and centrifugation.

The time delay allows the eluent fluid to advance across the interior surfaces until it halts before adding more eluent fluid. As such, adding of eluent fluid can be controlled to avoid superfluous amounts, which would lengthen subsequent evaporation.

In one embodiment, a robotic machine performs at least the step of placing the collecting device in the receptacle, adding the eluent, and removing the collecting device from the receptacle. The robotic machine allows for automation in the breath sample preparation, streamlining the process and enabling high reproducibility. Additionally, high-precision metering and delivery of the eluent fluid is achieved.

In one embodiment, the eluent fluid may comprise alcohol (ethanol, methanol, etc.), glycol of isopropanol, ethylene, ammonium bicarbonate, detergent, or a mixture thereof. The choice of eluent fluid depends on the type of analyte to be detected and/or the type of analysis to be carried out on the breath sample.

In one embodiment, the method may comprise separating the components of an eluate in the receptacle using liquid chromatography and identifying the separated components of the eluate using mass spectrometry. Advantageously, the steps of separating and identifying the components of the eluate may be carried out using a liquid chromatography mass spectrometer, LC/MS, or a liquid chromatography tandem mass spectrometer, LC/MS/MS. Mass spectrometry analysis is particularly suitable for detection of exogenous compounds such as drugs.

In one embodiment, the method may comprise amplifying at least one nucleic acid sequence present in an eluate in the receptacle using polymerase chain reaction, PCR. PCR testing is particularly suitable for detection of biomarkers in body tissue or fluid on a molecular or cellular level and pathogens such as virus, fungi or bacteria.

According to a second aspect of the present disclosure, there is provided a system for preparing a breath sample from a subject for analysis. The breath sample is collected using a device for collecting aerosol particles in an exhaled airflow which comprises a housing and at least four transverse baffles. The housing extends in a longitudinal direction between a first end with an inlet and a second end with an outlet. Additionally, the housing has an inner cross-section with a transverse width defined by an inner circumferential wall of the housing. The at least four transverse baffles are arranged at a distance from each other and extend substantially perpendicular to the inner wall, partly covering the inner cross section of the housing. The transverse baffles protrude from opposite sides of the inner wall of the housing to create a zigzag-shaped flow path from the inlet to the outlet.

The system comprises a robotic machine, a shaker, and a centrifuge. The robotic machine is configured to place the collecting device in a receptacle, add an eluent fluid onto the collecting device in the receptacle in a quantity smaller than or equal to 20 µl up to a total volume of approximately 30-60 µl, and repeat the adding step one or more times, wherein each adding step is performed with a predetermined time delay. The shaker is configured to agitate the receptable with the collecting device and the centrifuge to centrifuge the receptacle with the collecting device. As such, the system may be arranged to carry out the method for breath sample preparation according to the first aspect.

In one embodiment, the system may comprise a liquid chromatography apparatus to separate the components of the eluate and a mass spectrometer to analyze the separated components of the eluate. Furthermore, the robotic machine may be configured to transfer the receptacle to the shaker, the centrifuge concentrator, the liquid chromatography apparatus and/or the mass spectrometer.

In one embodiment, the receptacle has a substantially cylindrical shape, where a bottom section of the receptacle comprises a portion with a smaller diameter forming a compartment at the bottom of the receptacle. Additionally, the compartment has a volume smaller than or equal to 50 µl. With any one of these two alternatives, the receptacle may comprise a plurality of spacer elements arranged adjacent the compartment at the bottom of the receptacle and configured to support the collecting device. The shape of the receptacle is adapted to receive the collecting device such that it rests above the bottom compartment. After agitation and centrifugation, the resulting eluate is allowed to collect in the bottom compartment, separated from the collecting device. Preferably, the volume of the bottom compartment is dimensioned to contain about 90 % of the added eluent fluid, e.g., about 45 µl in the case where 50 µl of eluent fluid is added.

### Brief Description of Drawings

The present disclosure is now described, by way of example, with reference to the accompanying drawings, in which:
Fig. 1A is a cross-sectional view of a collecting device illustrating the working principle of collecting aerosol particles in exhaled breath;
Figs. 1B and 1C illustrate end views of the collecting device of Fig. 1A;
Fig. 1D is an end view of an alternative collecting device;
Fig. 2A is a cross-sectional view of a collecting device according to one embodiment of the present disclosure;
Fig. 2B is a cross-sectional view of a collecting device according to another embodiment of the present disclosure;
Fig. 3 is a view illustrating handling of a collecting device and receptacle according to one embodiment of the present disclosure; and
Figs. 4A-4D are cross-sectional views of a collecting device and receptacle illustrating different stages during delivery of eluent fluid in a method according to one embodiment of the present disclosure.

### Description of Embodiments

In the following, a detailed description of device according to the present disclosure is presented. In the drawing figures, like reference numerals designate identical or corresponding elements throughout the several figures. It will be appreciated that these figures are for illustration only and do not in any way restrict the scope of the present disclosure.

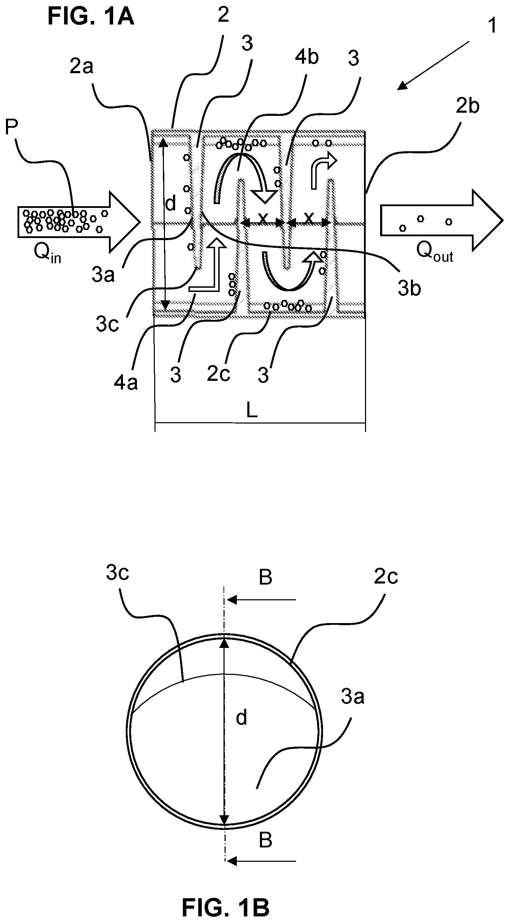

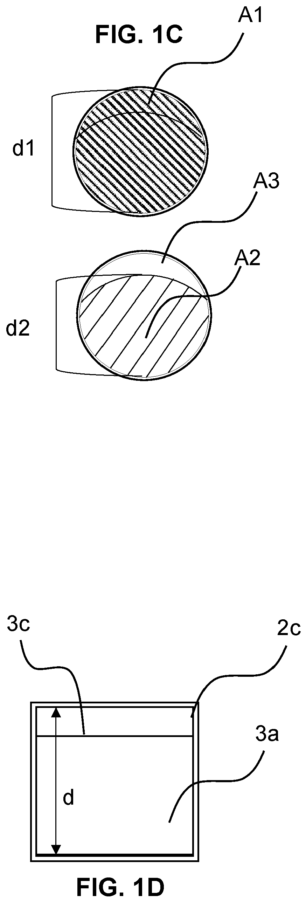

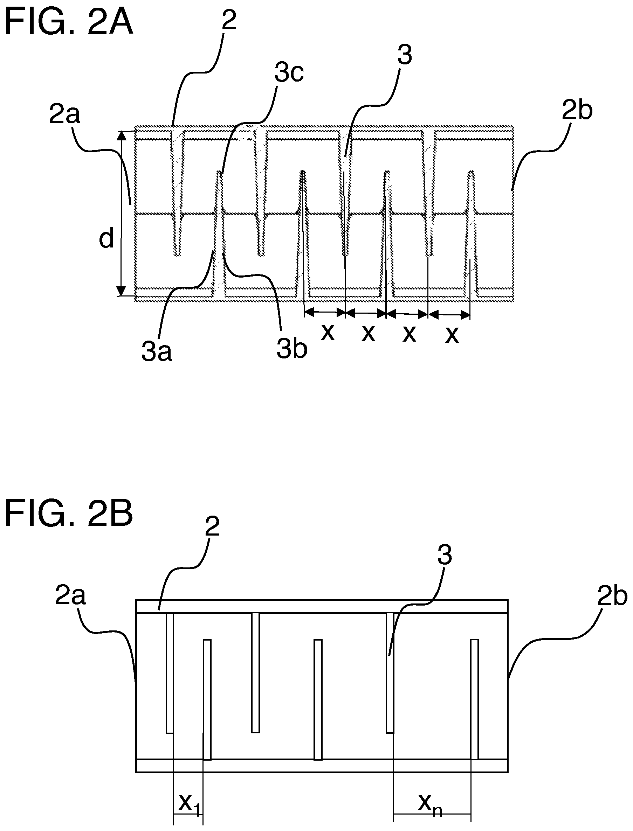

Figs. 1A-1D and 2A-2B disclose a device 1 for collection particles in exhaled breath. Fig. 1A is a cross-sectional view taken at cut B-B in Fig. 1B and shows the inside of the collecting device 1.

The collecting device 1 comprises a housing 2 having a length L in a longitudinal extension direction between a first end 2a with an inlet and a second end 2b with an outlet. The inlet is arranged to receive an exhaled airflow Qᵢₙ comprising aerosol particles P from a subject, such as for example a person, and the outlet is arranged to transmit the exhaled airflow Qₒᵤₜ from the collecting device 1. Thus, the exhaled air is arranged to flow in a direction from the inlet to the outlet. The housing 2 has an inner cross-sectional area defined by an inner circumferential wall 2c of the housing 2. In the embodiment shown in Figs. 1A-1C the housing has an elongated cylindrical shape with a length L and a circular cross-section, i.e. the cross-sectional area is defined by the transverse width equal to the inner diameter d of the housing 2. Fig. 1D discloses an alternative device where the housing 2 has a rectangular cross-section and the cross-sectional area is defined by the height and transverse width d of the housing. Other cross-sectional area shapes are also possible, but the cross-sectional area is always defined by the transverse width d which is equal to the distance between opposite inner walls 2c of the housing.

The outer diameter of the housing is in one embodiment of such a dimension that it can easily be fitted into a standard size test tube. More specifically, it may have a diameter between 8 and 30 mm, preferably between 10 and 20 mm. Said inner cross-sectional area is therefore slightly less than the area given by the outer diameter, depending on the thickness of the housing wall 2c. Therefore, said cross-sectional area may be between 20 mm² and 615 mm², preferably between 50 and 250 mm², most preferably between 70 and 90 mm². Comparably, said distance d between the inner walls 2c of the housing may be between 5 and 28 mm, preferably between 8 and 18 mm, most preferably between 9,5 and 10,5 mm.

At least four transverse baffles 3 in the form of partition walls are arranged to extend from the inner wall 2c in a substantially perpendicular direction, thus substantially perpendicular to the initial direction of the exhaled airflow when exiting the subject's mouth. Each transverse baffle 3 has a first surface 3a facing the airflow, an opposite second surface 3b and a peripheral edge or free end 3c. The first and second surface 3a and 3b each have a surface area smaller than the inner cross-sectional area of the housing 2. Thus, the transverse baffles have a surface area partly covering the inner cross-sectional area of the housing 2. In different embodiments the transverse baffles have a surface area covering 50-95%, preferably 60-85%, more preferably 65-80% of the inner cross-sectional area of the housing 2.

The transverse baffles 3 protrude from opposite sides of the inner wall 2c of the housing 2. Thus, the baffles create opposite openings 4a, 4b between the transverse baffles 3 and the housing inner wall 2c leaving an opening area equal to the difference between the inner cross-sectional area of the housing 2 and the surface area of the transverse baffle 3.

Said transverse baffles 3 are arranged to create a zigzag or labyrinth-shaped flow path having a cross-sectional flow area from said inlet to said outlet. When the airflow collides with a surface substantially perpendicular to the airflow, the flow is diverted in a direction parallel to said surface. Said diversion of the airflow separates the heavier particles P in the exhaled air from the air itself. The heavier particles P continue in the original flow direction and collide with the transverse baffles 3 or the inner wall 2c of the housing 2, while the air changes direction and follows the zigzag-shaped flow path. The longer distance the airflows and the more and larger direction changes the airflow is forced to do, the larger amount of particles is separated from the air and collected in the collecting device 1. Further, a direction change also creates a turbulent flow during which the particles are more easily separated from the air. A turbulent airflow also increases the impact frequency between the particles and the surfaces of the walls of the collecting device 1, thus increasing the amount of airborne particles P attaching to the surfaces. As a result, the outflow Qₒᵤₜ out of the collecting device 1 comprises less particles P than the inflow Qᵢₙ into the collecting device 1.

A person is only able to exhale with a certain maximum flow rate Qᵢₙ. At a certain counter pressure from the device the velum of the person closes and exhalation is impossible. The pressure difference over the device must therefore not be too high. However, a certain inflow Qᵢₙ and pressure difference is necessary to create the certain conditions with a high enough flow velocity to separate the particles from the airflow. It is therefore important to design the device to have a certain flow path cross-sectional flow area which is defined by a first cross-sectional flow area, defined by the opening area between the transverse baffles 3 and the inner wall 2c of the housing 2 and a second cross-sectional flow area delimited by the transverse baffles 3 and the inner diameter of the housing 2. The parameters defining the second cross-sectional flow area are the specific extension length L of the housing, the transverse width d of the housing 2 or inner diameter in the case of a cylindrical housing, and the number of transverse baffles 3 of the collecting device 1, more particularly, the distance x between the transverse baffles 3. The opening area mentioned above is preferably within the interval of 10 mm² - 25 mm², said extension length L between 10 and 70 mm and the number of transverse baffles 3 between four and fourteen. The first cross-sectional flow area is in one embodiment smaller than the second cross-sectional flow area. This relationship increases the acceleration of the air flowing past the peripheral edge 3c of the transverse baffle 3.

In the embodiment shown for example in Fig. 2A, the number of transverse baffles 3 are eight, the length L approximately 22 mm and the opening area is approximately 20 mm². Thus, in this embodiment the wall surface area A2 covers approximately 75% of the total inner cross-sectional area.

In order to increase the flow area, it is in one embodiment possible to arrange more than one device parallel to each other in an additional outer housing or holder as known from WO 2017/091134 A1, thus decreasing the total flow resistance.

The transverse baffles 3 may be separated from each other with a certain distance x depending on the maximum allowed pressure difference over the device. The distance x depends on the length L of the device and the number of transverse baffles 3. However, in order to create the certain conditions with a high enough flow velocity to separate the aerosol particles from the airflow, the distance x between at least two opposite transverse baffles 3 is always smaller than the distance d between the inner walls of the housing. In one embodiment, shown in Figs. 1a and 2a, the distance x is constant. Alternatively, the distance between adjacent transverse baffles 3 increases in the direction from the first end 2a towards the second end 2b of the collecting device 1, as shown in Fig. 2B. With an increasing distance between the transverse baffles 3 the flow velocity through the collecting device 1 can be substantially maintained. The second cross-sectional flow area increases towards the outlet 2b due to the increasing distance between the transverse baffles 3 while the first cross-sectional flow area equal to the opening area between the transverse baffles 3 and the inner wall 2c is kept constant. However, it is also possible to gradually increase the opening area in the direction from the first end 2a towards the second end 2b of the collecting device 1. This will further decrease the flow resistance in the device and contribute to a maintained flow velocity. In one embodiment, the relation between the first flow path cross-sectional flow area and the second flow path cross-sectional flow area is kept substantially constant throughout the entire length L of the device.

The device is in one embodiment made of a non-absorbent material, for example polymer materials such as for example polypropylene (PP), polyvinylidene fluoride (PVDF), polytetrafluoroethylene (PTFE), fluorinated ethylene propylene (FEP) or other non-absorbent, preferably, polymer materials. On a non-absorbent material the particles may attach but are easily washed off when a later analyzing step is performed. The washing off may be performed in a test tube filled with an amount of test fluid enough to cover the entire length L of the housing.

In order to increase the surface area on which the particles P are collected, in one embodiment the inner wall 2c of the housing 2 has a rough surface structure. The rough structure may be adapted to the size of the particles to be collected. In other words, if the aerosol particles to be collected have a diameter of 0.1 and 2.0 µm, the inner wall 2c may preferably be provided or covered with cavities of approximately the same size. The surface may also be machined to have protrusions distanced by approximately the same distance as the diameter of the particles. Said rough structure may also for example be a spark or electro eroded surface with a surface roughness Ra from 0.1 micron to up to 12.5 micron. Said possible surface roughness value also depend on the draft angle on the surface to be eroded in relation to the tool producing the eroded surface. With a larger draft angle, a larger surface roughness is possible to create.

It is also possible to further increase the surface area by providing all surfaces of the device, both inner and outer with a rough structure. Different surface roughness values are possible on different surfaces of the collecting device 1.

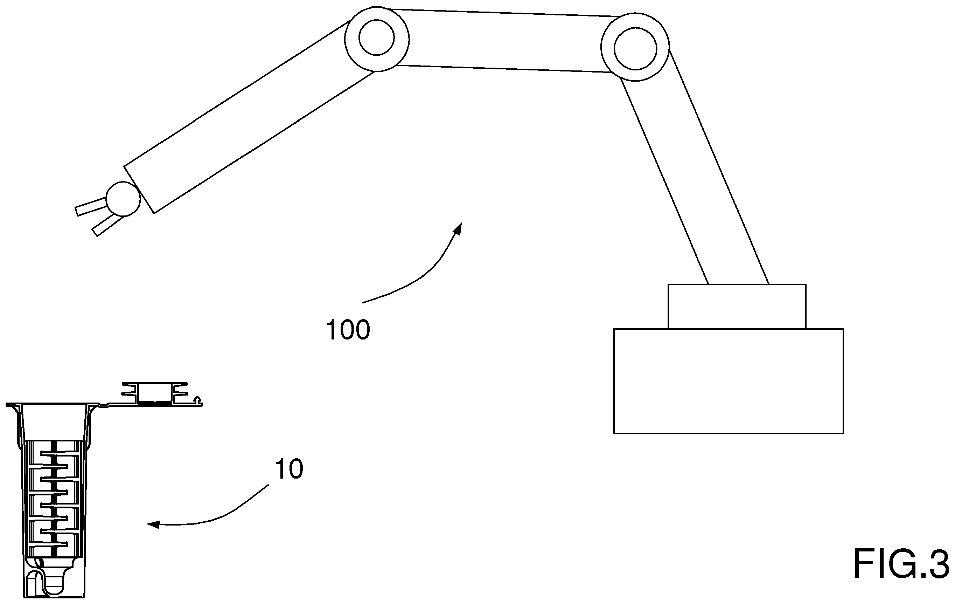

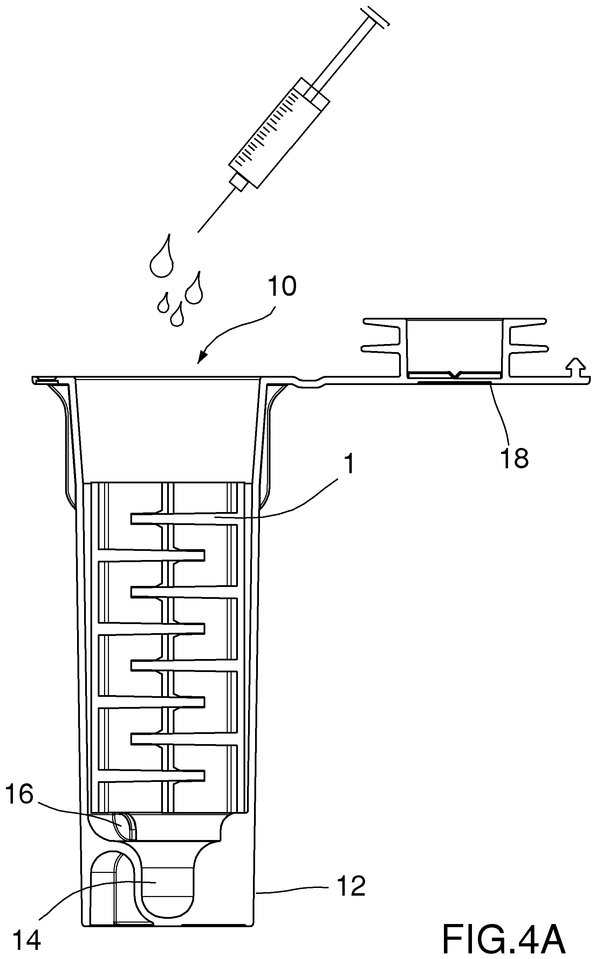

Referring now to Fig. 3 and Figs. 4A-4D, a receptacle 10 to be used in a method for preparing a breath sample according to the present disclosure is shown. The receptacle 10 has a substantially cylindrical shape, wherein a bottom section 12 of the receptacle 10 comprises a portion with a smaller diameter forming a compartment 14 at the bottom of the receptacle 10. The compartment 14 has a volume of about 50 µl, but may be larger or smaller, depending on the application.

The receptacle 10 may further comprise a plurality of spacer elements 16 arranged adjacent the compartment 14 at the bottom of the receptacle 10 and configured to support the collecting device 1 as shown in Fig. 3. This feature aids in separating the collecting device 1 from the eluate to be accumulated in the compartment 14. The receptacle 10 may further comprise a lid 18 connected to the top of the receptacle by means of a living hinge. The lid 18 is used to close the receptacle 10, e.g., during agitation and/or centrifugation. Alternatively, the receptacle may be closed by a separate stopper (not shown). As will be further discussed below, the different steps of handling the receptacle 10 and the collecting device 1 may be carried out using a robotic machine 100.

Referring now to Figs. 4A-4D, which show cross-sectional views of a collecting device in a receptacle 10, a method for preparing a breath sample for analysis according to the present disclosure will be described. A breath sample from a subject has been collected using the collecting device 1. The collecting device 1 is then placed in a suitable receptacle 10 for extracting the collected particles P through elution, as shown in Fig. 3. In the case of a collecting device 1 shaped like an elongated cylinder, the receptacle 10 may be a test tube or vial or similar. Optionally, the receptacle 10 may be placed on a stand or similar for increased stability.

An eluent fluid is added in a quantity that is smaller or equal to 20 µl, such as e.g., 10 µl. The eluent fluid is delivered onto the device in the receptacle 10 by means of a device capable of accurately delivering quantities of liquid smaller or equal to 20 µl. Such devices include a micropipette, a syringe, or any suitable dispensing arrangement. The micropipette tip or the needle of the micro syringe containing the eluent fluid is then introduced into the receptacle 10 adjacent the collecting device, and the eluent fluid is delivered to the interior of the housing 2 of the collecting device 1, as shown in Fig. 4A.

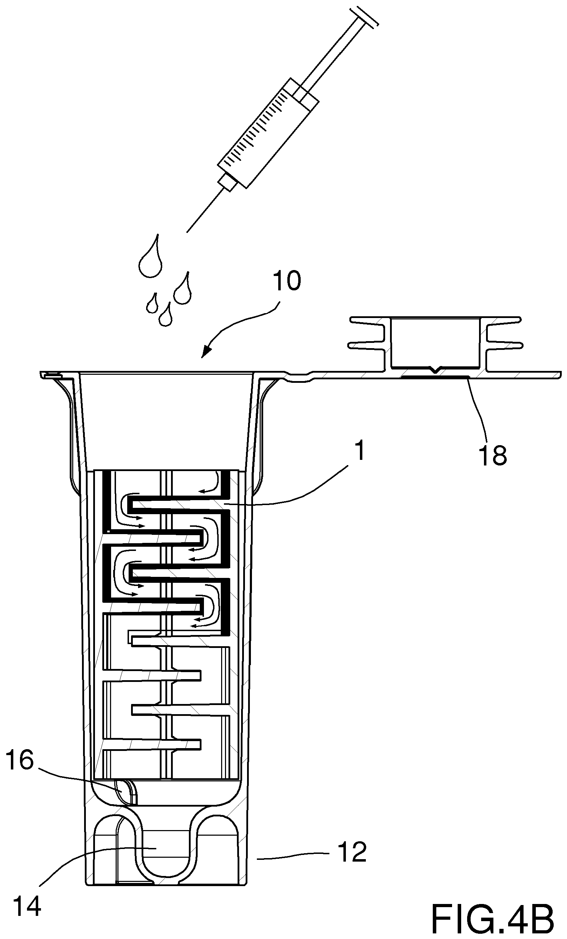

Referring now to Fig. 4B, the collecting device 1 and receptacle 10 is shown some time after the addition of the first amount of eluent fluid. As may be seen, the eluent fluid has moved along the interior surfaces of the collecting device 1, i.e., the inner walls 2c and the transverse baffles 3, advancing a portion of the length of the housing.

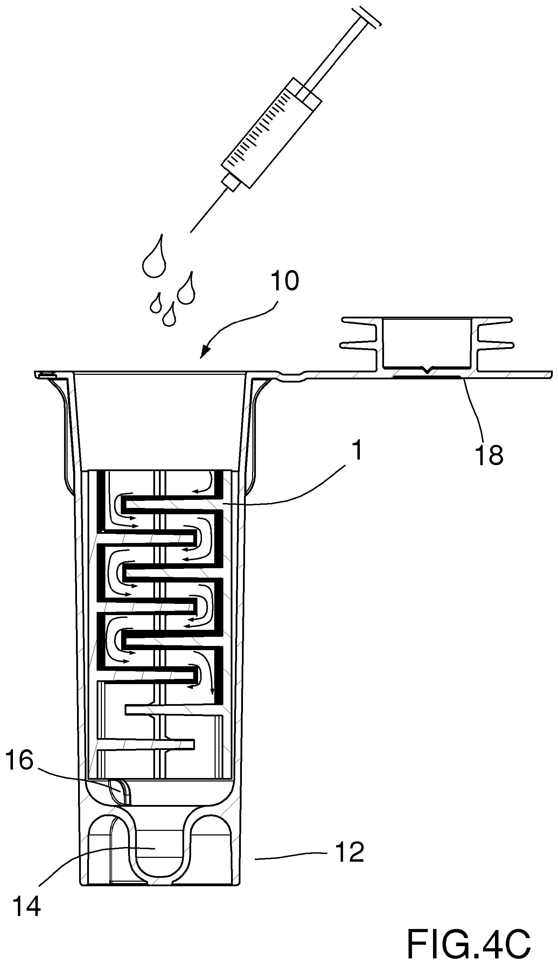

The effect of the eluent fluid is to wash the collected particles P from the interior surface of the housing 2 as it flows towards of the distal end of the collecting device, adjacent the bottom of the receptacle 10, due to gravity. However, due to the small amount (about 10-20 µl) of eluent fluid added, the collecting device 1 will not be inundated with eluent fluid. Instead, the eluent fluid will creep along the partition walls of the collecting device, but only a certain distance, as shown in Fig. 4C. It is believed that the eluent fluid interacts with the collected aerosol particles consisting mainly of surfactant originating from the alveoli of the lungs. This interaction causes the eluent fluid to advance slowly across the surfaces of the collecting device, loosening the particles in the process.

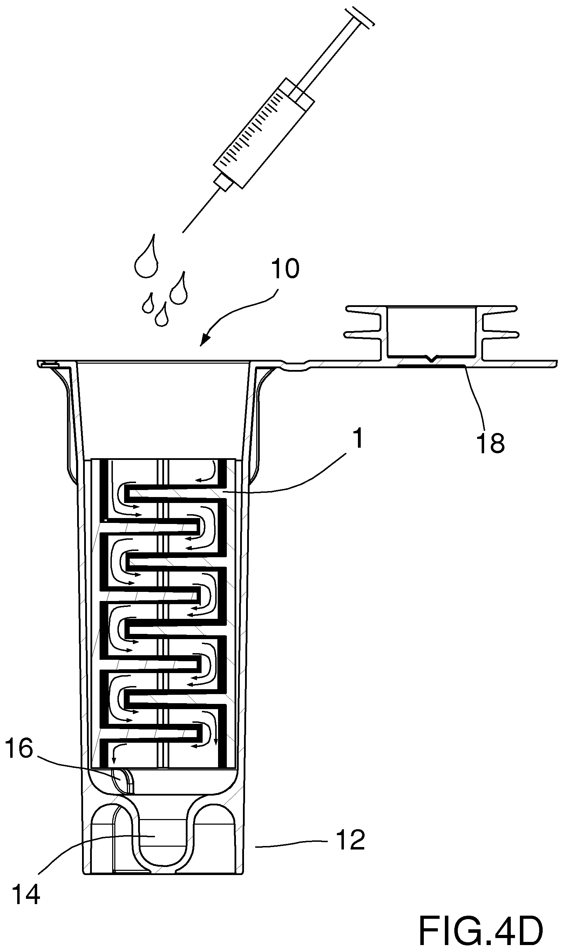

The step of introducing eluent fluid is then repeated several times, preferably at least one to five times. With each adding step, the eluent fluid continues to flow slowly downwards along the interior surfaces of the collecting device 1. Preferably, the addition of eluent fluid is repeated until the eluent fluid has reached the distal end of the collecting device, covering all the surfaces thereof, as shown in Fig. 4D. Each adding step is performed with a predetermined time delay. This delay may range from about 5-20 seconds up to 1-2 minutes. The delay may be adapted to the advancement of the eluent fluid along the interior surfaces of the collecting device, such that each subsequent adding step is performed only after advancement of eluent fluid slows down or ceases. Such a delay is advantageous because it allows for control of the required amount of eluent fluid to be added. In one embodiment, the adding of eluent fluid is repeated until the eluent fluid reaches a distal end of the collecting device, i.e., opposite the proximal end where eluent fluid is added.

In one embodiment, a total of approximately 50 µl of eluent fluid may be added to the device in amounts of about 10 µl during the eluent fluid addition process. This amount of eluent fluid is substantially lower than the 1.5 ml which is typically required using conventional sample preparation methods. The reduced amount of eluent fluid added in accordance with the method of the present disclosure allows for significant cost savings due to the high cost of eluents.

Additionally, with the reduced amount of eluent fluid, the amount of resulting eluate will be reduced which leads to a significant reduction in evaporation time. Compared to conventional sample preparation methods requiring up to 45-60 minutes to allow for concentration of the sample through evaporation, the method according to the present disclosure may reduce the sample preparation time to as low as 5 minutes. Consequently, the throughput of prepared breath samples may be increased as much as 12-fold compared to conventional techniques. This makes the method advantageous in a lab environment where a large number of breath samples are to be analyzed. The reduced time required for breath sample preparation also makes the method according to the present disclosure highly suitable for situations where quick analysis is desired, such as roadside or workplace testing for drugs or alcohol.

As an additional significant advantage, the accumulated eluate will have a higher concentration of aerosol particles compared to a method where a higher amount of eluent fluid is used. Surprisingly and advantageously, the use of repeatedly added volumes of eluent fluid of up to 20 µl, to a total volume of approximately 30-60 µl, preferably about 40-50 µl, allows for recovery of up to 90% of the collected particles in the collecting device 1. In one embodiment, the amount of eluent fluid added is selected in accordance with the subsequent analysis to be performed. For instance, the amount of eluent fluid may be adapted such that the resulting eluate volume corresponds to the required input volume of a liquid chromatography apparatus, thus obviating the need for concentration/evaporation.

The receptacle 10, e.g. a test tube or vial, is then placed in a shaker for agitating the collecting device. Agitating the collective device in contact with the eluent fluid promotes loosening and dispersion of the collected particles P from the interior surface of the housing, and flow of the eluent fluid and collected particles P towards the bottom of the receptacle 10. Any suitable shaker or vortex mixer designed for use with test tubes or vials may be used.

Once the agitating step is completed, the receptacle 10 is then placed in a centrifuge and centrifuged at about 1200-2000 RPM for approximately 3-5 minutes, so that the eluent fluid containing the collected particles P is pushed towards and accumulates at the bottom of the receptacle 10. Preferably, the centrifuge comprises a pivotable holder which allows the receptacle 10 to pivot to a substantially horizontal position during centrifugation, with the bottom of the test tube or vial facing outward.

The collecting device 1 is then removed from the receptacle 10 and the eluent fluid containing collected particles P is ready for analysis. Optionally, a step of concentration may be carried out, in which the eluate is evaporated, thereby increasing the concentration of analytes in the eluate.

In one embodiment, at least the steps of placing the collecting device in the receptacle 10, adding the eluent, and/or removing the collecting device from the receptacle 10 are performed by a robotic machine 100. A robotic machine allows for faster processing of the breath sample through automation. A robotic machine also does not require training to perform these tasks, and, furthermore, may have a higher accuracy rate and reproducibility than a human. The robotic machine may also be programmed to efficiently prepare several breath samples at the same time.

Once the eluent fluid containing collected particles P has been collected on the bottom of the receptacle 10, the components of the eluate may be separated into components using liquid chromatography and the separated components analyzed using mass spectrometry. As mentioned above, one significant advantage of micro elution lies in the fact that the eluent fluid may not need to be evaporated, thus saving both time and the costs attributable to evaporated, and thus wasted, eluent fluid.

The steps of separating and identifying the components of the eluate are carried out using a liquid chromatography mass spectrometer, LC/MS, or a liquid chromatography tandem mass spectrometer, LC/MS/MS. Using an LC/MS, or an LC/MS/MS is advantageous because it can offer both screening and identification at the same time, along with multi-component analysis and better sensitivity, such as lower cutoffs and longer detection times.

The eluent fluid may comprise alcohol (ethanol, methanol, etc.), glycol of isopropanol, ethylene, ammonium bicarbonate, detergent, or a mixture thereof. The choice of eluent fluid depends on the type of analyte to be detected and/or the type of analysis to be carried out on the breath sample. For instance, alcohol is detrimental to certain methods of analysis such as PCR testing. On the other hand, when the analyte to be tested is a biomarker, e.g., on a cellular level, alcohol aids in lysis of the cell membrane thereby facilitating subsequent analysis. In such a case, a mixture of eluent fluids including alcohol may be used, whereby the alcohol is allowed to evaporate during concentration of the eluate.

The preparation of a breath sample using the method described above can be used e.g., in drug or alcohol testing of a breath sample from a subject. This drug or alcohol testing can be performed outside a clinical laboratory, for example in a workplace environment or in roadside testing.

A system for preparing a breath sample for drug testing, where the breath sample has been collected using a device for collecting aerosol particles in an exhaled airflow, as previously described, comprises a robotic machine. The robotic machine is configured to place the collecting device in a receptacle 10. Next the robotic machine adds an eluent fluid onto the collecting device in the receptacle 10 in a quantity smaller than or equal to 20 µl and repeats the adding step one or more times. Once the adding step is completed, the robotic machine places the receptacle 10 in a shaker to agitate the contents of the shaker. The shaker may be a vortex mixer or vortexer. The robotic machine then places the receptacle 10 in a centrifuge which centrifuges the receptacle 10 and its contents, e.g., at 1200-2000 RPM for approximately 3-5 minutes.

The system may comprise a liquid chromatography apparatus arranged to separate the components of the eluate and a mass spectrometer arranged to analyze the separated components of the eluate. Advantageously the robotic machine is further configured to transfer the receptacle 10 to the shaker, the centrifuge concentrator, the liquid chromatography apparatus and/or the mass spectrometer.

By means of such a system, roadside or workplace testing can be completed in approximately 5-10 minutes (about 5 minutes to prepare the breath sample and about 5 minutes to analyze the breath sample), instead of the regular 90 minutes. During analysis (5 minutes) of a first breath sample, a second breath sample may be prepared (5 min) such that the second breath sample is ready for analysis when analysis of the first breath sample is completed, thereby considerably shortening the time required for subsequent analyses. Thus, the system and method according to the present disclosure enables a high throughput for analysis of a large number of breath samples.

Additionally, the components of the system may be sized so as to fit within a vehicle such as a car or van, thus achieving a mobile laboratory for roadside testing. Preferably, the system is arranged in a vehicle in such a way that the components are sealed or out of reach to a user to comply with regulatory requirements. The system is then fully automated and arranged to receive a portable sampling device as disclosed in WO 2017/091134 A1, extract a collecting device therefrom by means of the robotic machine, and subsequently perform the steps of the method according to the present disclosure.

Embodiments of a method for preparing a breath sample for analysis and a system for preparing a breath sample from a subject for analysis according to the present disclosure have been described. However, the person skilled in the art realizes that this can be varied within the scope of the appended claims.

## Claims

1. A method for preparing a breath sample for analysis, wherein the breath sample has been collected using a device (1) for collecting aerosol particles in an exhaled airflow, the device comprising:
a housing (2) extending in a longitudinal direction between a first end (2a) with an inlet and a second end (2b) with an outlet, and an inner cross-section defined by an inner circumferential wall (2c) of the housing, wherein the cross-section exhibits a transverse width (d); and
at least four transverse baffles (3), arranged at a distance from each other and extending in a direction substantially perpendicular to the inner wall, partly covering the inner cross section of the housing, wherein the transverse baffles protrude from opposite sides of the inner wall of the housing to create a zigzag-shaped flow path from the inlet to the outlet;
wherein the method comprises the steps:
placing the collecting device (1) in a receptacle (10);
adding an eluent fluid onto the collecting device (1) in the receptacle (10);
agitating the receptable using a shaker;
centrifuging the receptacle (10) using a centrifuge; and
removing the collecting device (1) from the receptacle (10),
**characterised in that** the eluent fluid is added in a quantity smaller than or equal to 20 µl, and the adding step is repeated one or more times up to a total volume of approximately 30-60 wherein each adding step is performed with a predetermined time delay.

2. The method according to claim 1, wherein at least the step of placing the collecting device (1) in the receptacle (10), adding the eluent, and/or removing the collecting device (1) from the receptacle (10) are performed by a robotic machine.

3. The method according to any one of the preceding claims, wherein the eluent fluid comprises alcohol (ethanol, methanol, etc.), glycol of isopropanol, ethylene, ammonium bicarbonate, detergent, or a mixture thereof.

4. The method according to any one of the preceding claims, further comprising:
separating the components of an eluate in the receptacle (10) using liquid chromatography, and
identifying the separated components of the eluate using mass spectrometry.

5. The method according to claim 4, wherein the steps of separating and identifying the components of the eluate are carried out using a liquid chromatography mass spectrometer, LC/MS, or a liquid chromatography tandem mass spectrometer, LC/MS/MS.

6. The method according to any one of claims 1-3, further comprising:
amplifying at least one nucleic acid sequence present in an eluate in the receptacle (10) using polymerase chain reaction, PCR.

7. A system for preparing a breath sample for analysis, wherein the breath sample has been collected using a device (1) for collecting aerosol particles in an exhaled airflow, the device comprising:
a housing (2) extending in a longitudinal direction between a first end (2a) with an inlet and a second end (2b) with an outlet, and an inner cross-section defined by an inner circumferential wall (2c) of the housing, wherein the cross-section exhibits a transverse width (d); and
at least four transverse baffles (3), arranged at a distance from each other and
extending in a direction substantially perpendicular to the inner wall, partly covering the inner cross section of the housing, wherein the transverse baffles protrude from opposite sides of the inner wall of the housing to create a zigzag-shaped flow path from the inlet to the outlet;
wherein the system comprises:
a robotic machine configured to:
place the collecting device (1) in a receptacle (10), and
add an eluent fluid onto the collecting device (1) in the receptacle (10);
a shaker configured to agitate the receptable with the collecting device (1); and
a centrifuge configured to centrifuge the receptacle (10) with the collecting device (1),
**characterised in that** robotic machine is configured to add the eluent fluid in a quantity smaller than or equal to 20 µl, and to repeat the adding step one or more times up to a total volume of approximately 30-60 µl, wherein each adding step is performed with a predetermined time delay.

8. The system according to claim 7, further comprising a liquid chromatography apparatus arranged to separate the components of the eluate; and a mass spectrometer arranged to analyze the separated components of the eluate.

9. The system according to claim 8, wherein the robotic machine is further configured to transfer the receptacle (10) to the shaker, the centrifuge concentrator, the liquid chromatography apparatus and/or the mass spectrometer.

10. The system according to any one of claims 7-9, wherein the receptacle (10) has a substantially cylindrical shape, and wherein a bottom section (12) of the receptacle (10) comprises a portion with a smaller diameter forming a compartment (14) at the bottom of the receptacle (10).

11. The system according to claim 10, wherein the compartment (14) has a volume smaller than or equal to 50 µl.

12. The system according to claim 10 or 11, wherein the receptacle (10) comprises a plurality of spacer elements (16) arranged adjacent the compartment (14) at the bottom of the receptacle (10) and configured to support the collecting device (1).

## Patentansprüche

1. Verfahren zur Vorbereitung einer Atemprobe zur Analyse, wobei die Atemprobe unter Verwendung einer Vorrichtung (1) zum Sammeln von Aerosolpartikeln in einem Ausatemluftstrom gesammelt worden ist, wobei die Vorrichtung Folgendes umfasst:
ein Gehäuse (2), das sich in einer Längsrichtung zwischen einem ersten Ende (2a) mit einem Einlass und einem zweiten Ende (2b) mit einem Auslass erstreckt, und einen inneren Querschnitt, der durch eine innere Umfangswand (2c) des Gehäuses definiert ist, wobei der Querschnitt eine Querbreite (d) aufweist; und
mindestens vier quer verlaufende Prallbleche (3), die in einem Abstand voneinander angeordnet sind und sich in einer Richtung erstrecken, die im Wesentlichen senkrecht zur inneren Wand verläuft, wobei sie den inneren Querschnitt des Gehäuses teilweise abdecken, wobei die quer verlaufenden Prallbleche von gegenüberliegenden Seiten der inneren Wand des Gehäuses vorstehen, um einen zickzackförmigen Strömungsweg vom Einlass zum Auslass zu erzeugen;
wobei das Verfahren die folgenden Schritte umfasst:
Platzieren der Sammelvorrichtung (1) in einem Behälter (10);
Zugeben einer Elutionsflüssigkeit auf die Sammelvorrichtung (1) in dem Behälter (10);
Schütteln des Behälters mittels eines Schüttlers;
Zentrifugieren des Behälters (10) mittels einer Zentrifuge; und
Entfernen der Sammelvorrichtung (1) aus dem Behälter (10),
**dadurch gekennzeichnet, dass** die Elutionsflüssigkeit in einer Menge kleiner als oder gleich 20 µl zugegeben wird und der Zugabeschritt ein oder mehrere Male bis zu einem Gesamtvolumen von ungefähr 30-60 µl wiederholt wird,
wobei jeder Zugabeschritt mit einer vorbestimmten Zeitverzögerung durchgeführt wird.

2. Verfahren nach Anspruch 1, wobei zumindest der Schritt des Platzierens der Sammelvorrichtung (1) in dem Behälter (10), des Zugebens des Eluenten und/oder des Entfernens der Sammelvorrichtung (1) aus dem Behälter (10) von einer Robotermaschine durchgeführt werden.

3. Verfahren nach einem der vorstehenden Ansprüche, wobei die Elutionsflüssigkeit Alkohol (Ethanol, Methanol usw.), Glykol aus Isopropanol, Ethylen, Ammoniumbicarbonat, Reinigungsmittel, oder eine Mischung davon umfasst.

4. Verfahren nach einem der vorstehenden Ansprüche, weiter umfassend:
Trennen der Komponenten eines Eluats in dem Behälter (10) mittels Flüssigkeitschromatographie, und
Identifizieren der getrennten Komponenten des Eluats mittels Massenspektrometrie.

5. Verfahren nach Anspruch 4, wobei die Schritte des Trennens und Identifizierens der Komponenten des Eluats mittels eines Flüssigkeitschromatographie-Massenspektrometers, LC/MS, oder eines Flüssigkeitschromatographie-Tandem-Massenspektrometers, LC/MS/MS, durchgeführt werden.

6. Verfahren nach einem der Ansprüche 1-3, weiter umfassend:
Amplifizieren von mindestens einer in einem Eluat in dem Behälter (10) vorhandenen Nukleinsäuresequenz mittels Polymerase-Kettenreaktion, PCR.

7. System zur Vorbereitung einer Atemprobe zur Analyse, wobei die Atemprobe unter Verwendung einer Vorrichtung (1) zum Sammeln von Aerosolpartikeln in einem Ausatemluftstrom gesammelt worden ist,
wobei die Vorrichtung Folgendes umfasst:
ein Gehäuse (2), das sich in einer Längsrichtung zwischen einem ersten Ende (2a) mit einem Einlass und einem zweiten Ende (2b) mit einem Auslass erstreckt, und
einen inneren Querschnitt, der durch eine innere Umfangswand (2c) des Gehäuses definiert ist, wobei der Querschnitt eine Querbreite (d) aufweist; und
mindestens vier quer verlaufende Prallbleche (3), die in einem Abstand voneinander angeordnet sind und
sich in einer Richtung erstrecken, die im Wesentlichen senkrecht zur inneren Wand verläuft, wobei sie den inneren Querschnitt des Gehäuses teilweise abdecken,
wobei die quer verlaufenden Prallbleche von gegenüberliegenden Seiten der inneren Wand des Gehäuses vorstehen, um einen zickzackförmigen Strömungsweg vom Einlass zum Auslass zu erzeugen;
wobei das System Folgendes umfasst:
eine Robotermaschine, die dazu ausgebildet ist:
die Sammelvorrichtung (1) in einem Behälter (10) zu platzieren, und
eine Elutionsflüssigkeit auf die Sammelvorrichtung (1) in dem Behälter (10) zuzugeben;
einen Schüttler, der dazu ausgebildet ist, den Behälter mit der Sammelvorrichtung (1) zu schütteln; und
eine Zentrifuge, die dazu ausgebildet ist, den Behälter (10) mit der Sammelvorrichtung (1) zu zentrifugieren,
**dadurch gekennzeichnet, dass** die Robotermaschine dazu ausgebildet ist, die Elutionsflüssigkeit in einer Menge kleiner als oder gleich 20 µl zuzugeben und den Zugabeschritt ein oder mehrere Male bis zu einem Gesamtvolumen von ungefähr 30-60 µl zu wiederholen,
wobei jeder Zugabeschritt mit einer vorbestimmten Zeitverzögerung durchgeführt wird.

8. System nach Anspruch 7, weiter umfassend eine Einrichtung zur Flüssigkeitschromatographie, die angeordnet ist, die Komponenten des Eluats zu trennen; und ein Massenspektrometer, das angeordnet ist, die getrennten Komponenten des Eluats zu analysieren.

9. System nach Anspruch 8, wobei die Robotermaschine weiter dazu ausgebildet ist, den Behälter (10) zu dem Schüttler, dem Zentrifugenkonzentrator, der Einrichtung zur Flüssigkeitschromatographie und/oder dem Massenspektrometer zu überführen.

10. System nach einem der Ansprüche 7-9, wobei der Behälter (10) eine im Wesentlichen zylindrische Form aufweist, und wobei ein Bodenabschnitt (12) des Behälters (10) einen Abschnitt mit einem kleineren Durchmesser umfasst, der eine Kammer (14) am Boden des Behälters (10) bildet.

11. System nach Anspruch 10, wobei die Kammer (14) ein Volumen kleiner als oder gleich 50 µl aufweist.

12. System nach Anspruch 10 oder 11, wobei der Behälter (10) eine Vielzahl von Abstandshalterelementen (16) umfasst, die benachbart zu der Kammer (14) am Boden des Behälters (10) angeordnet und dazu ausgebildet sind, die Sammelvorrichtung (1) zu stützen.

## Revendications

1. Procédé de préparation d'un échantillon d'air expiré pour analyse, dans lequel l'échantillon d'air expiré a été recueilli en utilisant un dispositif (1) de collecte de particules d'aérosol dans un flux d'air expiré, le dispositif comprenant :
un logement (2) s'étendant dans une direction longitudinale entre une première extrémité (2a) dotée d'une entrée et une seconde extrémité (2b) dotée d'une sortie, et une section transversale interne définie par une paroi circonférentielle interne (2c) du logement, dans lequel la section transversale présente une largeur transversale (d) ; et
au moins quatre déflecteurs transversaux (3), agencés à une certaine distance les uns des autres et s'étendant dans une direction sensiblement perpendiculaire à la paroi interne, couvrant partiellement la section transversale interne du logement, dans lequel les déflecteurs transversaux font saillie depuis des côtés opposés de la paroi interne du logement pour créer un trajet d'écoulement en zigzag de l'entrée à la sortie ;
dans lequel le procédé comprend les étapes suivantes :
le placement du dispositif (1) de collecte dans un récipient (10) ;
l'ajout d'un fluide éluant sur le dispositif (1) de collecte dans le récipient (10) ;
l'agitation du récipient en utilisant un agitateur ;
la centrifugation du récipient (10) en utilisant une centrifugeuse ; et
le retrait du dispositif (1) de collecte du récipient (10),
**caractérisé en ce que** le fluide éluant est ajouté en une quantité inférieure ou égale à 20 µl, et l'étape d'ajout est répétée une ou plusieurs fois jusqu'à atteindre un volume total d'environ 30-60 µl,
dans lequel chaque étape d'ajout est réalisée après un délai prédéterminé.

2. Procédé selon la revendication 1, dans lequel au moins l'étape de placement du dispositif (1) de collecte dans le récipient (10), d'ajout de l'éluant, et/ou de retrait du dispositif (1) de collecte du récipient (10) est réalisée par une machine robotisée.

3. Procédé selon l'une quelconque des revendications précédentes, dans lequel le fluide éluant comprend de l'alcool (éthanol, méthanol, etc.), du glycol d'isopropanol, de l'éthylène, du bicarbonate d'ammonium, un détergent, ou un mélange de ceux-ci.

4. Procédé selon l'une quelconque des revendications précédentes, comprenant en outre :
la séparation des composants d'un éluat dans le récipient (10) par chromatographie en phase liquide, et
l'identification des composants séparés de l'éluat par spectrométrie de masse.

5. Procédé selon la revendication 4, dans lequel les étapes de séparation et d'identification des composants de l'éluat sont réalisées en utilisant un spectromètre de masse couplé à la chromatographie en phase liquide, LC/MS, ou un spectromètre de masse en tandem couplé à une chromatographie en phase liquide, LC/MS/MS.

6. Procédé selon l'une quelconque des revendications 1 à 3, comprenant en outre :
l'amplification d'au moins une séquence d'acide nucléique présente dans un éluat dans le récipient (10) en utilisant une réaction en chaîne par polymérase, PCR.

7. Système de préparation d'un échantillon d'air expiré pour analyse, dans lequel l'échantillon d'air expiré a été recueilli en utilisant un dispositif (1) de collecte de particules d'aérosol dans un flux d'air expiré,
le dispositif comprenant :
un logement (2) s'étendant dans une direction longitudinale entre une première extrémité (2a) dotée d'une entrée et une seconde extrémité (2b) dotée d'une sortie,
et une section transversale interne définie par une paroi circonférentielle interne (2c) du logement, dans lequel la section transversale présente une largeur transversale (d) ; et
au moins quatre déflecteurs transversaux (3), agencés à une certaine distance les uns des autres et
s'étendant dans une direction sensiblement perpendiculaire à la paroi interne, couvrant partiellement la section transversale interne du logement, dans lequel les déflecteurs transversaux font saillie depuis des côtés opposés de la paroi interne du logement pour créer un trajet d'écoulement en zigzag de l'entrée à la sortie ;
dans lequel le système comprend :
une machine robotisée configurée pour :
placer le dispositif (1) de collecte dans un récipient (10), et
ajouter un fluide éluant sur le dispositif (1) de collecte dans le récipient (10) ;
un agitateur configuré pour agiter le récipient avec le dispositif (1) de collecte ; et
une centrifugeuse configurée pour centrifuger le récipient (10) avec le dispositif (1) de collecte,
**caractérisé en ce que** la machine robotisée est configurée pour ajouter le fluide éluant en une quantité inférieure ou égale à 20 µl, et pour répéter l'étape d'ajout une ou plusieurs fois jusqu'à atteindre un volume total d'environ 30-60 µl,
dans lequel chaque étape d'ajout est réalisée après un délai prédéterminé.

8. Système selon la revendication 7, comprenant en outre un appareil de chromatographie en phase liquide conçu pour séparer les composants de l'éluat ; et un spectromètre de masse conçu pour analyser les composants séparés de l'éluat.

9. Système selon la revendication 8, dans lequel la machine robotisée est en outre configurée pour transférer le récipient (10) vers l'agitateur, le concentrateur centrifuge, l'appareil de chromatographie en phase liquide et/ou le spectromètre de masse.

10. Système selon l'une quelconque des revendications 7 à 9, dans lequel le récipient (10) présente une forme sensiblement cylindrique, et dans lequel une section inférieure (12) du récipient (10) comprend une partie présentant un diamètre plus petit formant un compartiment (14) au fond du récipient (10).

11. Système selon la revendication 10, dans lequel le compartiment (14) présente un volume inférieur ou égal à 50 µl.

12. Système selon la revendication 10 ou la revendication 11, dans lequel le récipient (10) comprend une pluralité d'éléments d'espacement (16) agencés de manière adjacente au compartiment (14) au fond du récipient (10) et configurés pour soutenir le dispositif (1) de collecte.
